# EUROPEAN PATENT APPLICATION

(11) **EP 1 736 462 A1**
(43) Date of publication of application: **27.12.2006**
(21) Application number: 05013394.1
(22) Date of filing: 22.06.2005
(51) Int. Cl.: C07C 67/52, C07C 67/60, C07C 69/76

(54) **Recovery of optically active tartaric acid resolving agents**

(71) Applicant: SANDOZ AG, 4056 Basel (CH)
(72) Inventor: Albert, Martin, A-6262 Bruck am Ziller (AT); Berger, Andreas, A-6330 Kufstein (AT)
(74) Representative: Dietz, Jörg-Reimar

(57) **Abstract**

A process for the recovery of substituted tartaric acid resolving agents from resolution mother liquors or isolated diastereomeric salts, characterized in that the diastereomeric salt of an enantiamerically enriched amine and the substituted tartaric acid derivative in an organic solvent is treated with a base, extracting said substituted tartaric acid derivative into an aqueous phase, adding an acid to the separated aqueous phase while monitoring the pH-value, precipitating mono salts of substituted tartaric acid derivatives, isolating said mono salts, and liberating the substituted tartaric acid derivative by an acidic treatment.

## Description

This invention relates to a novel process for the recovery of optically active tartaric acid resolving agents from diastereomeric salts. The tartaric acid derivatives are obtained in very good to excellent yields and sufficient optical purity to be reused in the corresponding resolution process.

Optically active acids, such as (+)-di-*O,O'*-acyl tartaric acid or (-)-di-*O,O'*-acyl tartaric acid derivatives, are frequently employed for the synthesis of optically active compounds containing an arnino group. The separation of the enantiomeric amines is accomplished by salt formation with the resolving agent. One of the diastereomeric salts, preferentially the salt containing the amine with the desired configuration, crystallizes due to a lower solubility. The required optically active amine can be regenerated by basification of the diastereomeric salt. Thereby, the tartaric acid derivative is obtained as a di-salt in aqueous solution.

Previous to this invention, methods used to recover substituted tartaric acid resolving agents from this aqueous phase have been found to give unsatisfying yields and insufficient purity. The purification of the resolving agent has to be done by crystallization in order to remove products which are formed due to base induced side reactions such as transesterification or deesterification. Previous recovery methods are based on the extraction of diacyl tartaric acids into water immiscible or poorly water miscible organic solvent by addition of an acid with subsequent crystallization from another solvent (Jacques, J.; Collet, A.; Wilen, S.H. Enantiomers, Racemates and Resolutions, John Wiley & Sons, New York, 1981). Such solvent exchange Operations are connected to extended preparative work.

WO 03/042132 is related to a process which is based on a crystallization of di-*O,O'*-acyl tartaric acid derivatives from the aqueous phase in the presence of a mineral acid and an organic co-solvent. By using C₁ to C₁₀ alcohols as co-solvent the free tartaric acid derivatives crystallize as a solvate with the corresponding alcohol. However, the mother liquor consists of a mixture of water with an organic solvent, which again complicates large scale operations.

According to WO 04/063141 the crystallization af di-*O,O'*-toluoyl tartaric acid is carried out directly from the diastereomeric salt in water, in the presence of a mineral acid, and crystalline di-*O,O'*-toluoyl tartaric acid. However, the regeneration is performed in the presence of the amine and a strong mineral acid. The amine has to be stable under these conditions. Depeading on the nature of the amine co-precipitation with the tartaric acid can also occur. Therefore, this process is only applicable to a limited set of amines.

Examples of resolving agents used in such resolution processes include (+)-di-*O,O'*-toluoyl-D-tartaric acid, (-)-di-*O,O'*-toluoyl-L-tartaric acid, (+)-di-*O,O'*-benzoyl-d-tartaric acid, (-)-di-*O*,*O*'-benzoyl-L-tartaric acid, (+)-di-*O,O'*-anisoyl-D-tartaric acid, (-)-di-*O,O'*-anisoyl-L-tartaric acid, or mixture of them. Examples of such structures are shown in figure 1.

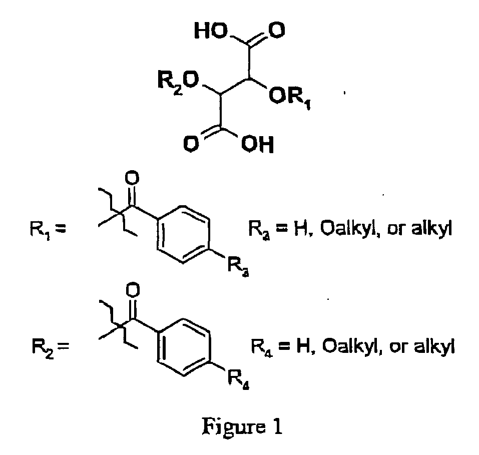

The present invention is directed to a novel process for the recovery of substituted tartaric acid resolving agents from resolution mother liquors or isolated diastereomeric salts. The process is characterized in that the diastereomeric salt of an enantiomerically enriched amine and the substituted tartaric acid derivative in an organic solvent is treated with a base, extracting said substituted tartaric acid derivative into an aqueous phase, adding an acid to the separated aqueous phase while monitoring the pH-value, precipitating mono salts of substituted tartaric acid derivatives, isolating said mono salts, and liberating the substituted tartaric acid derivative by an acidic treatment
This process avoids the use of an additional solvent for the crystallization and yields sufficiently pure substituted tartaric acid derivatives in good to excellent yields.

The diastereomeric salt consisting of a chiral amine and a substituted tartaric acid derivative is dissolved or suspended in an organic solvent immiscible or only poorly miscible with water, optionally in admixture with water. A base is added in order to set free the amine moiety. Alternatively, said diastereomeric salt is dissolved or suspended in water, treated with base, and an organic solvent immiscible or only poorly miscible with water is added. Partitioning results in an organic phase containing the chiral amine and an aqueous phase containing the di-anion of the substituted tartaric acid derivative. Surprisingly it was found that the substituted tartaric acid derivative can be precipitated from the aqueous phase as a substituted tartaric acid mono salt by careful addition of the corresponding quantity of mineral acid (scheme 1). Due to the controlled and mild reaction conditions no side reactions such as deesterification, transesterification or racemisation are observed. The mono salt of the substituted tartaric acid derivative is obtained in a crystalline form of suitable purity. Further purification of the isolated crystals may be effected by washing or recrystallization, if necessary. This mono salt is transformed into the free di-acid by reaction by an acidic ion exchanger in an organic solvent or organic solvent / water mixture. Separation of the ion exchanger by filtration gives a solution of the substituted tartaric acid derivative in the organic solvent or organic solvent / water mixture. Alternatively, the transformation of the substituted tartaric acid mono salt to the corresponding free acid can be performed using a mineral acid in water and a poorly water miscible or immiscible solvent. A solution of the liberated substituted tartaric acid derivative in a poorly water miscible or immiscible solvent is thereby obtained which is provided as a solution ready to use in subsequent resolution processes.

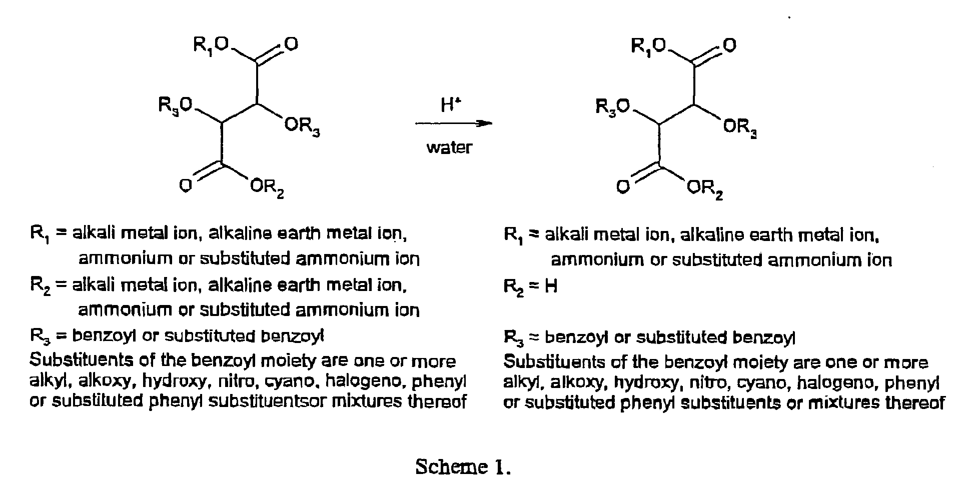

The mono salts as intermediates of the inventive process have, according to the base used, a cationic counter ion which is an alkali metal ion, an alkaline earth metal ion, an ammonium or optionally substituted ammonium ion. The acyl moiety is a benzoyl or substituted benzoyl. Substituents of the benzoyl moiety are one or more allcyl, preferably C₁ - C₄ alkyl, alkoxy, preferably C₁ - C₄ alkoxy, hydroxy, nitro, cyano, halogeno, phenyl or substituted phenyl substituents.

Resolution mother liquors typically comprise one or more organic solvents in addition to the resolving agent and the optically enriched amine. Typical organic solvents used for the resolving process are alcohols such as for instance 2-propanol, ketones such as for instance acetone, ethers such as for instance methyl *tert*-butyl ether, aromatic hydrocarbons, such as for instance toluene, or mixtures thereof. Preferably the organic solvent is only poorly miscible with water or water immiscible. Water miscible solvents such as 2-propanol need to be removed and replaced by a poorly water miscible or immiscible solvent, e.g. methyl *tert-*butyl ether or methylene chloride. After treatment with base, the salt is partitioned into an organic phase containing the chiral amine and an aqueous phase containing the di-anion of the substituted tartaric acid derivative.
The base used for setting free the amine moiety of the diastereomeric salt may be ammonia, substituted ammonia, a hydroxide, carbonate or hydrogen carbonate of an alkali or alkaline earth metal. Preferably the base is ammonia. The base can be added in the form of an aqueous solution.

The mineral acid used to obtain the mono salts of substituted tartaric acid derivatives may be any strong acid, such as hydrochloric, hydrobromic, sulphuric, nitric or phosphoric acid, preferably hydrochloric acid. The mineral acid is added in amounts sufficient to protonate only one of the carboxylic acid groups of the tartaric acid derivative. Unexpectedly, the substituted tartaric acid mono salt crystallizes in high yield and in a pure form suitable for further transformations. In order to achieve this, the pH-value of the aqueous phase has to be adjusted, according to the cationic counter ion present. If the counter ion is potassium, the pH value has to be adjusted to 2.0 - 5.0. If sodium is the counter ion, the pH value is adjusted to 2.0 - 4.0, or, if ammonium is the cationic counter ion, to 2.0 - 2.8, more preferably to 2,4 - 2.5.

The recovery of the substituted tartaric acid mono salt is typically performed at 5°C to 50°C, more preferably at 20°C to 25°C. The product is filtered off and dried, typically under reduced pressure at 25°C. Optionally, the wet product can be used directly for the subsequent steps. The substituted tartaric acid mono salt is obtained in >90% yield.

The transformation of the substituted tartaric acid mono salt into the free di-acid is done by reaction with an acidic ion exchanger in an organic solvent or organic solvent / water mixture or by a mineral acid in water and a poorly water miscible or immiscible solvent. The ion exchanger can be any acidic cation exchanger, which is sufficiently strong to protonate the substituted tartaric acid mono salt, such as for instance Amberlite IR-120. The mineral acid used to liberate the substituted tartaric acid derivative may be any strong acid, such as hydrochloric, hydrobromic, sulphuric, nitric or phosphoric acid, preferably hydrochloric acid.

A particular advantage of the present invention is that the tartaric acid derivative can be isolated in a pure form directly out of the aqueous phase without addition of a co-solvent. The protonation of the substituted tartaric acid mono salt can be performed in any solvent using an acidic ion exchanger thus giving a solution of the substituted tartaric acid in the required solvent.

The results obtained in resolution processes using as resolving agents recovered substituted tartaric acid derivatives prepared by the process of the present invention are equivalent to those obtained starting with previously unused substituted tartaric acid derivatives.

The process according to the present invention is particularly advantageous in the recovery of di-*O,O'*-toluoyl-tartartic acid from mother liquors and in the manufacture of optically pure 4-[(*S*)-4-*N,N*-Dimethylamino-1-(4-fluoro-phenyl)-1-hydroxy-butyl]-3-hydroxymethyl-benzonitrile, a key intermediate in the synthesis of escitalopram.

The invention is further described by reference to the following examples. These examples are provided for illustration purposes only and are not intended to be limiting the present invention in any way.

### EXAMPLES:

1) 40.09g of 4-[(*R*)-4-*N,N-*Dimethylamino-1-(4-fluoro-phenyl)-1-hydroxy-butyl]-3-hydroxymethyl-benzonitrile hemi-(+)-di-*O,O'*-toluoyl-D-tartaric acid (or the corresponding amount of a resolution mother liquor in a water immiscible solvent) are suspended in 400ml of methyl *tert*-butyl ether (or dichloromethane) and 280ml of H₂O. Addition of 13.1ml of aqueous 25% NH₃ (17.03g/mol, corresponds to 2.5 eq.) gives a homogenous solution after stirring for 10 minutes. The aqueous layer is washed with 120ml of methyl *tert*-butyl ether (or dichloromethane). The combined organic layer is dried over Na₂SO₄, filtered and concentrated under reduced pressure to give 4-[(*R*)-4-*N,N*-dimethylamino-1-(4-fluorophenyl)-1-hydroxy-butyl]-3-hydroxymethyl-benzonitrile.
   The pH of the aqueous phase is adjusted to 2.4 by addition of 170 ml 1M aqueous HCl. Stirring is continued for 5h. The mono-NH₄-salt of (+)-di-toluoyl-D-tartaric acid is collected by filtration (G3-suction filter, d=9.5cm) and washed with 50ml of H₂O. The white product is dried under reduced pressure (20mbar) for 15h at 25°C to give 12.95g of (+)-di-*O,O'*-toluoyl-D-tartaric acid mono-NH₄ salt (93.7% yield (assay: 93.4% of (+)-di-*O,O'*-toluoyl-D-tartaric acid; melting point 172-173°C; specific rotation (C=1, ethanol) +139°.
   ¹H-NMR (DMSO-d₆): δ: 2.36 (6H, 2x CH₃), 5.66 (2H, 2x CH), 7.29 (4H, aromatic protons), 7-84 (4H, aromatic protons), 7.0-9.0 (m, 4H);
   ¹³C-NMR (DMSO-d₆): δ: 167.9, 164.7, 143.5, 129.2, 129.0, 126.9, 71.8, 21.0).
   IR (Diamond ATR cell): wave number cm⁻¹ (intensity % T) 3255 (0.8), 3033 (0.85), 1703 (0.43), 1609 (0.62), 1577 (0.61), 1509 (0.72), 1409 (0.53), 1337 (0.69), 1266 (0.25), 1229 (0.42), 1211 (0.57), 1177 (0.42), 1122 (0.41), 1105 (0.2), 1021 (0.47), 954 (0.6), 933 (0.7), 860 (0.75), 838 (0.65), 786 (0.68), 753 (0.25), 703 (0.48), 636 (0.78).

### Liberation of (+)-di-O,O'-toluoyl-D-tartaric acid:

### Method A:

4.27g of (+)-di-*O,O'*-toluoyl-D-tartaric acid mono-NH₄ salt are suspended in 40ml of 2-propanol. 16.0g of Amberlite 1R 120 are washed with 40mL of 2-propanol and added to the suspension of (+)-di-*O,O'*-toluoyl-D-tartaric acid mono-NH₄ salt in 2-propanol. The mixture is stirred until the solution becomes clear. Removal of the ion exchanger by filtration and washing with 40ml of 2-propanol gives a solution of 4.05 g of (+)-di-*O,O'*-toluoyl-D-tartaruc acid in 2-propanol, which can be used directly for subsequent resolutions.

### Method B:

17.0g of (+)-di-*O,O'*-toluoyl-D-tartaric acid mono-NH₄ salt are suspended in 130ml of water and 130ml of dichloromethane. The pH of the corresponding suspension is adjusted to a pH <0.5 by addition of 6M aqueous HCL After phase separation the organic layer can directly be used for the subsequent resolutions.
2) The preparation of DTTA-Mono-potassium salt is performed according to example one, but with the exception to use 10% KOH instead of 25% NH₃ for the first neutralization. The pH of the aqueous phase is adjusted to 4,8 by addition of aqueous HCl. After stirring for additional 3h the mono-potassium-salt of (+)-di-toluoyl-D-tartaric acid is collected by filtration (G3-suction filter, d=9.5cm) and washed with H₂O. The white product is dried under reduced pressure (20mbar) for 15h at 25°C. (assay 87,1% DTTA, 8,6% potassium; 3% H2O) Liberation of free acid DTTA may be performed according to Method A or B.
3) The preparation of DTTA-Mono-sodium salt is performed according to example one, but with the exception to use 20% NaOH instead of 25% NH₃ for the first neutralization. The pH of the aqueous phase is adjusted to 3,7 by addition of aqueous HCl, After stirring for additional 3h the mono-sodium salt of (+)-di-toluoyl-D-tartaric acid is collected by filtration (G3-suction filter, d=9.5cm) and washed with H₂O. The white product is dried under reduced pressure (20mbar) for 15h at 25°C.
   Liberation of free acid DTTA may be performed according to Method A or B.

## Claims

1. A process for the recovery of substituted tartaric acid resolving agents from resolution mother liquors or isolated diastereomeric salts, **characterized in that** the diastereomeric salt of an enantiomerically enriched amine and the substituted tartaric acid derivative in an organic solvent is treated with a base, extracting said substituted tartaric acid derivative into an aqueous phase, adding an acid to the separated aqueous phase while monitoring the pH-value, precipitating mono salts of substituted tartaric acid derivatives, isolating said mono salts, and liberating the substituted tartaric acid derivative by an acidic treatment.

2. Compounds of the general formula

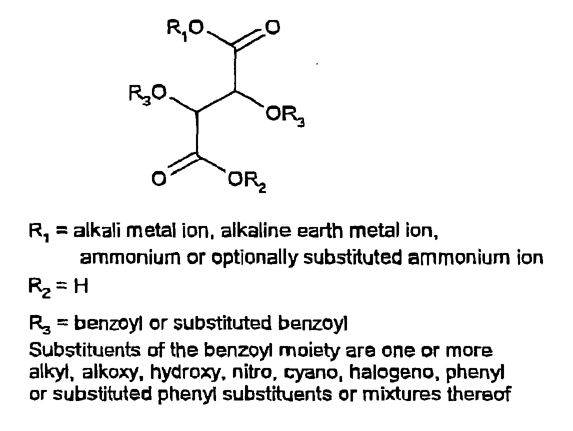

3. The process according to claim 1, wherein the base is a hydroxide, carbonate or hydrogen carbonate salt of an alkali or alkaline earth metal or ammonia or optionally substituted ammonia.

4. The process according to claim 1, wherein the pH value of the aqueous solution is adjusted to 2.0 to 5.0.

5. The process according to claim 1, wherein the pH value of the aqueous solution is adjusted to 2.0 to 4.0.

6. The process according to claim 1, wherein the pH value of the aqueous solution is adjusted to 2.0 to 2.8.

7. A process according to any of the preceding claims, wherein the pH value of the aqueous solution is adjusted to 2.4 to 2.5.

8. The process according to claim 1, wherein the acid used to obtain mono salts of substituted tartaric acid derivatives is a mineral acid such as hydrochloric acid, hydrobromic acid, sulfuric acid, nitric acid or phosphoric acid.

9. The process according to claim 1, wherein the acidic treatment of the substituted tartaric acid mono salt is performed by an acidic ion exchanger or a mineral acid to give the corresponding free substituted tartaric acid derivative.

10. The process according to claim 9, wherein the acidic treatment of the substituted tartaric acid mono salt is performed by an acidic ion exchanger or a mineral acid in an organic solvent or mixtures of an organic solvent and water.

11. The process according to claims 9 and 10, wherein the mineral acid used to liberate the substituted tartaric acid derivative is hydrochloric acid, hydrobromic acid, sulfuric acid, nitric acid or phosphoric acid.

12. The process according to claims 9 and 10, wherein the ion exchanger is an acidic cation exchanger.

13. The process according claims 9 and 10, wherein the ion exchanger is an Amberlite IR 120 cation exchanger.

14. The process according to claim 1, wherein the liberated substituted tartaric acid derivative is provided as a solution in a poorly water miscible or immiscible solvent ready to use in subsequent resolution processes.
